(19) **Europäisches Patentamt / European Patent Office / Office européen des brevets**

(11) **EP 1 833 855 B1**

(12) **EUROPEAN PATENT SPECIFICATION**

(45) Date of publication and mention
of the grant of the patent:
**17.12.2008 Bulletin 2008/51**

(51) Int Cl.:
*C08F 6/06* (2006.01)  *C08F 6/12* (2006.01)
*B01J 19/00* (2006.01)  *C08J 3/09* (2006.01)
*A61K 47/00* (2006.01)  *G02F 1/00* (2006.01)
*C12N 11/08* (2006.01)

(21) Application number: **05813295.2**

(22) Date of filing: **09.11.2005**

(86) International application number:
**PCT/IT2005/000653**

(87) International publication number:
**WO 2006/051572 (18.05.2006 Gazette 2006/20)**

(54) **METHOD FOR CONTROLLING THE DIMENSIONS AND THE MORPHOLOGY OF NANOSTRUCTURAL POLYMERIC MATERIALS, MATERIALS THEREBY OBTAINED AND USES THEREOF**

VERFAHREN ZUR STEUERUNG DER ABMESSUNGEN UND DER MORPHOLOGIE VON NANOSTRUKTURELLEN POLYMERMATIERIALIEN, DADURCH ERHALTENE MATERIALIEN UND VERWENDUNG DAVON

PROCEDE PERMETTANT DE REGLER LES DIMENSIONS ET LA MORPHOLOGIE DE MATERIAUX POLYMERES NANOSTRUCTURES, MATERIAUX AINSI OBTENUS ET LEURS UTILISATIONS

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.11.2004 IT RM20040555**

(43) Date of publication of application:
**19.09.2007 Bulletin 2007/38**

(73) Proprietor: **Universita'Degli Studi di Roma "La Sapienza"**
**00185 Roma (IT)**

(72) Inventors:
• **PALOCCI, Cleofe**
**Piazzale Aldo Moro, 5,**
**I-00185 (IT)**
• **RUSSO, Maria, Vittoria**
**Piazzale Aldo Moro, 5,**
**I-00185 (IT)**
• **BELSITO, Carmela, Maria, Angelica**
**Piazzale Aldo Moro, 5,**
**I-00185 (IT)**
• **CERNIA, Enrico**
**Piazzale Aldo Moro, 5,**
**I-00185 (IT)**

• **D'AMATO, Rosaria**
**Piazzale Aldo Moro, 5,**
**I-00185 (IT)**
• **FRATODDI, Ilaria**
**Piazzale Aldo Moro, 5,**
**I-00185 (IT)**
• **PANZAVOLTA, Fabrizio**
**Piazzale Aldo Moro, 5,**
**I-00185 (IT)**
• **SORO, Simonetta**
**Piazzale Aldo Moro, 5,**
**I-00185 (IT)**
• **VENDITTI, Iole**
**Piazzale Aldo Moro, 5,**
**I-00185 (IT)**

(74) Representative: **Capasso, Olga**
**De Simone & Partners S.p.A.**
**Via Vincenzo Bellini, 20**
**00198 Roma (IT)**

(56) References cited:
**WO-A-03/097015**      **WO-A-20/05040755**
**US-A- 5 948 470**      **US-A1- 2003 022 242**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- R. D'AMATO, L. MEDEI, I. VENDITTI, M.V. RUSSO, M. FALCONIERI: "Chemical synthesis of polyphenylacetylene nanospheres with controlled dimensions for photonic crystals" MATERIALS SCIENCE AND ENGINEERING C, vol. 23, 2003, pages 861-865, XP002369781 cited in the application

- M. MÜLLER, R. ZENTEL, T. MAKA, S. G. ROMANOV, C. M. SOTOMAYOR TORRES: "Dye-containing polymer beads as photonic crystals" CHEM. MATER., vol. 12, 2000, pages 2508-2512, XP002369782 cited in the application

**Description**

Introduction

The present invention relates to a method for controlling the dimensions and the morphology of nanostructured polymeric materials, materials thereby obtained and uses thereof in different industries.

Obtaining new materials with controllable dimensions and shape at sub-micrometric scale has opened new perspectives in many fields: from electronics and photoelectronics (carbon nanotubes, quantum dots, materials for photonic crystals, etc.) to the textile industry (carbon nanofibres), from pharmaceuticals (nanospheres and nano-capsules for drug delivery) to medicine (new diagnostic instruments), from fine and supramolecular chemistry (nanostructured catalysers, molecular machines) to the production of energy (new photovoltaic cells, new batteries) [1,2,3,4,5].

At the industrial level, the challenges related to the development of processes calling for nanometric control of the dimensions of the materials are a problem for the scale-up of nanotechnologies. Therefore, there is an evident need to provide economic and alternative methods for industrial production.

In this sense, interest for polymeric compound has greatly grown: their ease of processing, the extreme solubility in numerous organic solvents and the excellent non linear optic properties which many of them have, offer various application opportunities. The reason for their interest is to combine the lightness, low cost and ease of processing that are typical of polymers with characteristic properties of inorganic materials. Indeed, they are used for varnishes, glues, coatings, impact modifiers, molecule immobilisation and encapsulation, drug delivery, photonic crystals, materials for cosmetics. Nanostructured polymers are generally synthesised with emulsion polymerisation techniques entailing the use of emulsifiers, which constitute an additional cost and pollute the product. In the absence of emulsifiers, a drastic lowering of yields is obtained [6,7,8,9,10,11,12].

General description of the invention

The authors of the present invention have devised a method for controlling the dimensions and the morphology of polymers, that is innovative, simple and low cost. The method provides polymers with different morphology starting from polymers synthesised with any procedure, without the use of emulsifiers. The method exploits a physical barrier, exemplified in dialysis membranes or more in general in semi-permeable membranes that allow the passive transport of solutes, to slow down the mixing of a polymer solution with a non solvent of the polymer itself (the miscibility of the solvent and of the non solvent must be high, preferably complete), in which the dialysis bag containing the solution of the polymer is immersed. As a variant, able further to slow the process, the bag can be immersed in the same solvent present inside, whereto is slowly added the non solvent by dripping (e.g. by means of a peristaltic pump). The gradual mixing of the solvent and of the non solvent inside the bag causes the mixture that is being formed to be progressively less able to solubilise the polymer. The consequent increase in the interface tension pushes the molecules of the polymer to aggregate in spheroidal particles, whose shape allows to minimise the energy of the system, which then form a precipitate. The speed whereat the process takes place is the factor that most influences the morphology of the final product and it is determined by various parameters, among them the solvent - non-solvent pair, the rate of addition of the non solvent to the system and the temperature. The morphology of the particles is then determined by kinetic and thermodynamic factors [13]. From a thermodynamic viewpoint, the most important parameter is the free interface energy. When the precipitation process is slow enough, the morphology is not influenced by the kinetic parameters and the so-called equilibrium morphology is reached, i.e. the one that corresponds to the minimisation of the free energy of the system. With regard to the kinetic parameters, the ones with the greatest influence on particle morphology are: the diffusion and the phase rearrangement inside the particles themselves. The mobility of the polymer chains is restricted during their aggregation and hence phase separation and rearrangement can be slower than the aggregation rate.

In this case, kinetic factors prevail over thermodynamic ones and morphologies different from the spherical one are obtained, called non-equilibrium. The spherical morphology, reached when the thermodynamic factors prevail, is the equilibrium morphology [13].

Control over morphology is easily obtained acting on a few experimental parameters: choice of materials, concentrations, number and type of membranes, temperature. Polymer recovery is total and takes place by centrifuging and subsequent drying.

The advantages of the method, in addition to low costs and general validity, are manifold: work is conducted in bland conditions, avoiding the use of emulsifiers and co-stabilisers, thereby obtaining pure products and recovering the solvents by distillation. Moreover, the treatment allows to carry out the precipitation process simultaneously with that of molecule immobilisation (or similar processes) also with biological activity (enzymes, drugs, etc.) allowing the functionalisation of the polymer in order to obtain the specific characteristics for the use and/or the particular system of employment during the "structurisation" process on nano and microscopic scale.

In the invention, therefore, a new method for obtaining nanostructured polymeric systems is proposed. The method is based on a new use of the dialysis technique (currently used to purify various substance, also of polymeric nature) to induce nanostructured morphologies in polymers of different chemical nature. The polymers can belong both to the synthetic and the natural polymer class. Synthetic polymers belong to classes of polymers obtainable by polymerisation in stages (condensation, e.g. Poly(diethynylbiphenylditributylphosphinaPt(II) or through chain polymerisation (addition and opening of ring, e.g. Polyphenylacetylene, Polymethylmethacrylate, Polystyrene).

The polymer dissolved in a solvent is closed in a dialysis membrane and then put in contact with a non solvent in which it precipitates. After recovering the solid by centrifuging, chemical-physical tests (IR, UV-vis) are carried out to confirm the non degradation of the material, and SEM analysis is performed to study its morphology. The morphologies obtained vary from amorphous, to spongy, to tubular (non-equilibrium morphologies) to spheres of dimensions ranging between microns and nanometres, depending on the particular conditions of the process.

Detailed Description of the Invention

The object of the present invention therefore is a method for controlling the dimensions and the morphology of the nanostructure of at least one polymer, comprising the steps of:

- exposing, through a semi-permeable membrane that allows the passive transport of solutes, a solution of the polymer, pre-dissolved in a solvent able to dissolve the polymer in substantially complete fashion, to a non-solvent of the polymer, in which the solvent of the polymer is miscible, wherein the temperature, the v/v solvent / non-solvent ratio and the polymer concentration are so selected as to obtain the precipitate polymer in the desired nanostructure;
- separating the precipitate polymer from the solution.

In a preferred embodiment the semi-permeable membrane is an osmotic membrane, more preferably it is a dialysis membrane.

The semi-permeable membrane can be a natural or synthetic membrane, based on regenerated cellulose, cellulose derivatives, with hydrophobic or hydrophilic characteristics. The type of membrane is selected according to the chemical-physical characteristics both of the solvent/non-solvent pair and of the polymer, in such a way as to obtain the precipitate polymer in the desired nanostructure.

Preferably, the exposure is conducted between a solution of the polymer in a solvent able to dissolve the polymer in substantially complete fashion, and the same solvent of the polymer into which the non-solvent of the polymer is gradually added.

The polymers can be both natural and synthetic. Synthetic polymers can be obtained by polymerisation in stages (condensation, e.g. Poly(diethynylbiphenylditributylphosphinaPt(II)) or through chain polymerisation (addition and opening of ring, e.g. Polyphenylacetylene, Polymethylmethacrylate, Polystyrene).

In an embodiment, the solvent and the non-solvent belong to the groups: Alcohols, Ketones, Ethers, Amides, aliphatic, aromatic and chlorinated organic solvents, and $H_2O$. Preferably, the solvent and the non-solvent belong to the group: Acetone, Methanol, Ethanol, Tetrahydrofuran, Dimethylformamide, Dimethylsulphoxide, Acetonitrile, Toluene, Chloroform, Hexane, $H_2O$.

The person skilled in the art will select, according to the polymer and to the nanostructured morphology to be obtained, the following parameters: concentration of the polymer, solvent / non-solvent pair, solvent / non-solvent volume ratio, temperature of the dialysis process, number and type of the semi-permeable membranes (or of the physical barrier/ alternative porous septum), procedures for adding the non-solvent which then influence the speed of diffusion of the non-solvent in the solvent.

Examples of polymers and adequate but not limited solvent / non-solvent pairs are shown in Table 1.

Table 1

| Reaction | Solvent-non solvent | [Polymer] mg/ml | Polymer | Morphology |
|----------|---------------------|-----------------|---------|------------|
| D14 | DMF/MeOH | 0.5 | PPA | 5 |
| D 21 | DMF/MeOH | 10 | PPA | 4 |
| D 22 | DMF/$H_2O$ | 10 | PPA | 3 |
| D 3 | THF/$H_2O$ | 5 | PPA | 2 |
| D 2 | THF/$H_2O$ | 0.5 | PPA | 1 |
| D 27 | THF/$H_2O$ | 10 | PMMA | 5 |
| D 44 | DMF/MeOH | 10 | PS | 5 |

(continued)

| Reaction | Solvent-non solvent | [Polymer] mg/ml | Polymer | Morphology |
|---|---|---|---|---|
| D 58 | THF | 5 | PtDEBP38 | 2 |

The authors of the present invention have applied the use of nanostructured polymeric matrices in transporting molecules with carrier function for the immobilisation of lipolytic enzymes from GRAS (Generally Recommended As Safe) source widely used in industrial bio-transformations, for processes for resolving enantiomeric mixtures or for the synthesis of bulk chemicals. The results obtained show that:

1. The adsorption of fungal lipases, e.g. lipases from *Candida rugosa* (CRL) and bacterial, e.g. lipase from *Pseudomonas cepacia* (PCL) on nanostructured PS and PMMA enables to obtain stable bioconjugates.
2. Nanostructured polymeric materials have, in equal conditions, a high loading capacity with respect to amorphous polymers.
3. The residual activity of the lipases used adsorbed on polymeric nanospheres is rather high (see Tables 2-5), for conformational variations induced by the adsorption on the carrier able to stabilise the lipases themselves.
4. The adsorption of one of the lipases considered (CRL) on nanostructured PMMA confers a "Langmuir" fitting, index of a highly favourable adsorption.

These lipolytic enzymes supported on nanostructured carriers, having a better catalytic performance, can be employed in industrial processes of hydrolysis, synthesis of esters and amides in different industrial sectors of application.
The present invention shall now be described in some non limiting examples, in reference to the figures.
Fig. 1: structures of the used polymers.
Fig. 2: example of different PPA morphologies: morphology no. 1 cavernous type, morphology no. 2 spongy type, morphology no. 3 tubular type, morphology no. 4 spheres molten together, morphology no. 5 sphere type, the latter with luminescence properties.

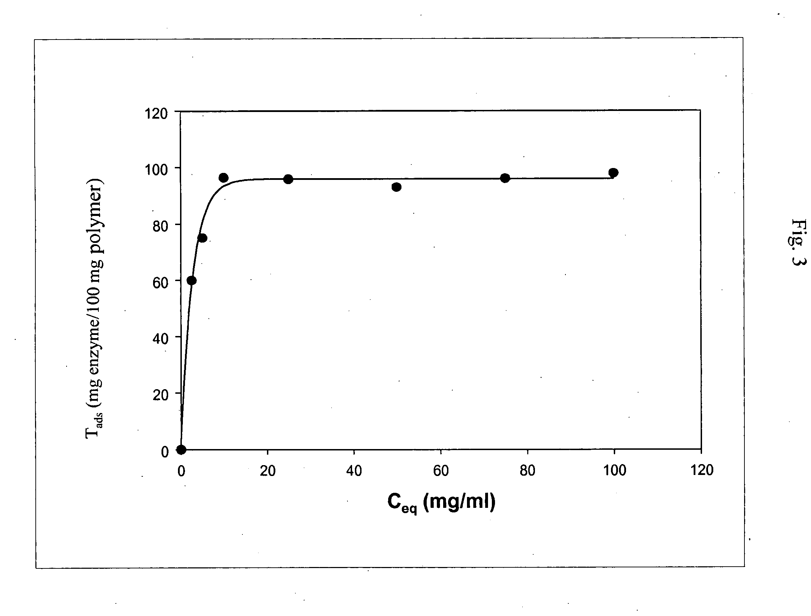

Fig. 3: adsorption isotherm of the CRL on PMMA nanostructured at 25°C.
Different polymers were used at different concentration, different solvents and mixtures, different dialysis membranes and different number of membranes, different nonsolvents, different temperature and agitation conditions and different ways and times of contact with the non-solvent (immersion and dripping). Some tests are shown in Table 2.

Table 2 - Tests conducted at ambient temperature on different polymers, from which different morphologies are obtained according to the selected conditions.

| Reaction | Solvent/non solvent | [PPA] mg/ml | Membrane | Solv / non solv * | Polymer | Morp |
|---|---|---|---|---|---|---|
| D 2 | THF / dist. $H_2O$ | 0.5 | single | 1/20 | PPA | 1 |
| D 3 | THF / dist. $H_2O$ | 5 | single | 1/40 | PPA | 2 |
| D 4 | DMF / dist. $H_2O$ | 0.5 | single | 1/20 | PPA | 3 |
| D 20 | DMF / hexane | 5 | double | 1/20 | PPA | 4 |
| D 7 | DMF / dist. $H_2O$ | 10 (saturated) | double | 1/20 | PPA | 5 |
| D 25 | DMF / dist. $H_2O$ | 10 | single | 1/20 | PMMA | 3 |
| D 26 | DMF / MeOH | 10 | single | 1/60 | PMMA | 4 |
| D 27 | THF / dist. $H_2O$ | 10 | single | 1/40 | PMMA | 5 |
| D 33 | DMF / dist. $H_2O$ | 5 | single | 1/20 | PMMA | 2 |
| D 34 | DMF / ethanol | 5 | single | 1/20 | PMMA | 3 |
| D 58 | THF / dist. $H_2O$ | 5 | single | 1/40 | PtDEBP382 | |

(continued)

| Reaction | Solvent/non solvent | [PPA] mg/ml | Membrane | Solv / non solv * | Polymer | Morp |
|---|---|---|---|---|---|---|
| D 59 | DMF / Acetone | 1 | single | 1/40 | PtDEBP382 | |
| D 41 | DMF / dist. H$_2$O | 10 | single | 1/20 | polystyrene | 4 |
| D 44 | DMF / Methanol | 10 | single | 1/20 | polystyrene | 5 |
| D 45 | DMF / ethanol | 10 | single | 1/20 | polystyrene | 4 |

Materials

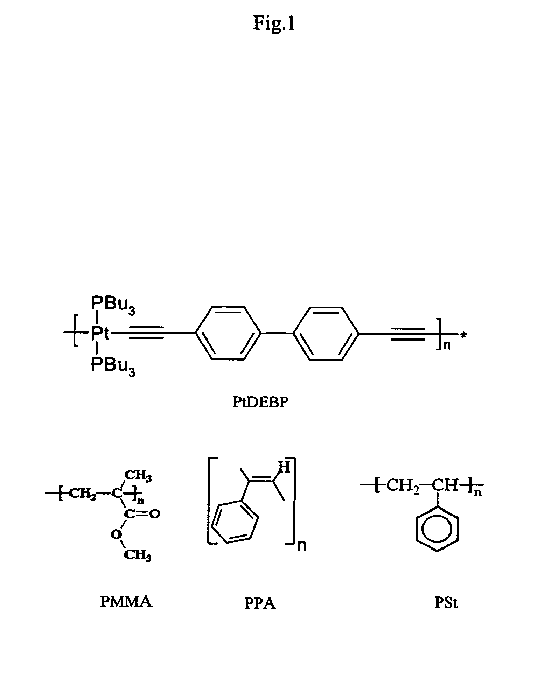

Figure 1 shows the structures of the used polymers, listed herein:

- Polyphenylacetylene (PPA), synthesised in accordance with [14];
- Poly(diethynylbiphenylditributylphosphinaPt(II)) (PtDEBP), synthesised in accordance with [15];
- Commercial Goodfellow polymethylmethacrylate (PMMA) (pure for analysis);
- Commercial Goodfellow polystyrene (PSt), (pure for analysis).

Solvents and non solvents, used pure or in mixtures, are of the Aldrich "pure for analysis" grade: Acetone, Methanol, Ethanol, Tetrahydrofuran, Dimethylformamide, Dimethylsulphoxide, Acetonitrile, Toluene, Chloroform, Hexane. Water is distilled water.

Membranes: Sigma cellulose membranes both normal - retention of cytochrome C - 12400 Da - >90% in 10 h - and high retention - retention of cytochrome C >99% in 10 h

Instrumentation

When the method is carried out at low temperature, a Haake GH (Fisons) cryostat was used. To recover the precipitated polymer as a result of the process, a model 121 R ALC PK refrigerated ultracentrifuge was used.

The chemical-physical analyses to verify that the polymer had not undergone degradation as a result of the process were carried out with conventional IR techniques (Perkin-Elmer 1700 FT IR instrument (4000-400 cmP$^-$1) and UV-vis spectrophotometer (Perkin Elmer lambda 5 / Varian Cary 100).

The analysis of the surfaces conducted with electron scanning microscopy was performed with a SEM-LEO1450VP on metallised samples.

Method

In order to produce polymers in the form of spheroidal particles in more simple fashion than emulsion polymerisation, the following polymers were considered by way of example: polyphenylacetylene (PPA), Poly(diethynylbiphenylditributylphosphinaPt(II)) (PtDEBP), Polymethylmethacrylate (PMMA), and Polystyrene (PSt). On these polymers, a post-synthesis precipitation treatment was devised, based on interface phenomena connected to the mixing of a polymer solution with a non-solvent thereof, exploiting the slow-down in the process obtained through the use of dialysis membranes as physical barriers able to hinder the mixing of the two liquids. Sigma cellulose membranes were used. The formation of solid particles by precipitation is also influenced by the selection of the solvent / non-solvent pair; however, the two liquids must be miscible together (the quantity of solvent used must be totally miscible with the non-solvent, present in ample excess). The different morphologies obtained were classified as follows:

- morphology no. 1: cavernous type;
- morphology no. 2: spongy type;
- morphology no. 3: tubular type;
- morphology no. 4: agglomerates of molten spheres;
- morphology no. 5: spheres with dimensions between 4 micron and 100 nanometres.

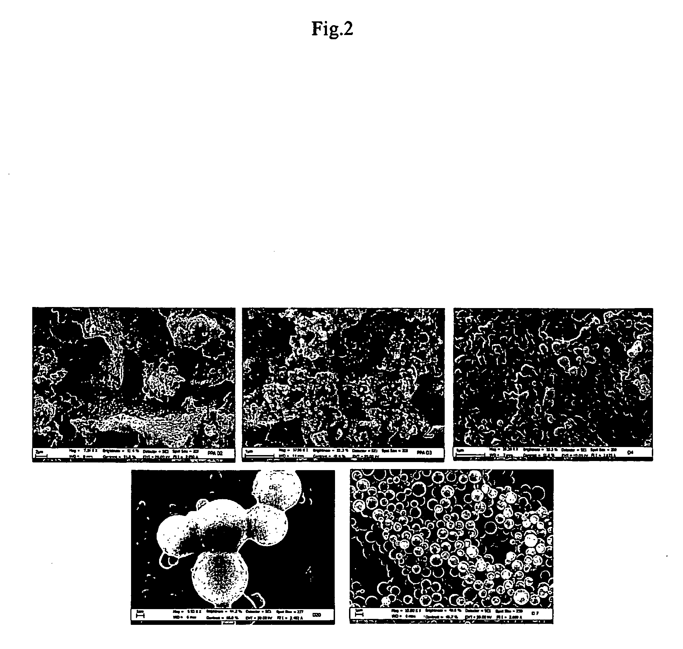

Fig. 2 shows for example different morphologies of PPA, which also has the characteristic of being a luminescent material.

The factor that most influences the final morphology of the polymer is probably represented by the speed of the process in which the polymer comes in contact with the non-solvent, during which the solvent molecules that surround the polymer are replaced with non-solvent molecules. In general, the morphology obtained is determined by kinetic and thermodynamic factors [13]: the spherical morphology is the equilibrium morphology, reached when thermodynamic factors are prevalent. The other morphologies are non-equilibrium morphologies. In the case of morphology no. 5, when polymer spheres are obtained, other aspects need to be considered: the dimensions of the particles obtained and polydispersion. Control of dimensions and of polydispersion becomes fundamental for example in case of applications in the field of optoelectronics, since optical properties strongly depend on the shape and dimension of the particles and on their packaging [11,16,17]. To have applications like photonic crystals in optical devices, the particles must have regular shape (generally spherical) and dimensions between 100 and 800 nm. High values of polydispersion entail the presence of particles of different dimensions, responsible for many defects in structures that self-assemble. Polydispersion was calculated using the following formula:

$[(d\mathrm{B}_{maxB} - d\mathrm{B}_{minB})/d\mathrm{B}_{avB}]$, where $d\mathrm{B}_{maxB}$, $d\mathrm{B}_{minB}$ and $d\mathrm{B}_{avB}$ are respectively the maximum, minimum and medium diameter of the polymer spheres.

It is necessary to stress that the measurements of the dimensions of the aggregates were conducted with the aid of the Scion Image software directly on the photos at the SEM and, therefore, it is necessary to take into account the uncertainty due to the statistical validity of the photographed sample.

Table 3 shows some data on dimensions and polydispersion of the polymer spheres obtained in different conditions.

Table 3 - Dimensions and polydispersion of polymers with morphology no. 5 (spheres) obtained in different conditions.

| Reaction | Solvent-non solvent | [PPA] mg/ml | membrane * | solv / non solv | Polymer | diameter (nm) | Polydispersion [(dmax - dmin)/dav] |
|---|---|---|---|---|---|---|---|
| D 15 | DMF-MeOH | 0.5 | single | 1/20 | PPA | 220-950 | 1.25 |
| D 52 | THF-$H_2O$ dist. | 0.5 | single | dripping | PPA | 1500- 5000 | 1.08 |
| D 19 | DMF-$H_2O$ dist. | 0.5 | double | 1/20 | PPA | 100-650 | 1.47 |
| D 27 | THF-$H_2O$ dist. | 10 | single | 1/40 | PMMA | 400-2000 | 1.33 |

Table 4 - Immobilisation of the lipase from *Pseudomonas cepacia* (PCL) on nanostructured polymethylmethacrylate (PMMA) (309 nm $\varnothing$)

| SAMPLE | ENZYMATIC CHARGE * |
|---|---|
| PCL on amorphous PMMA | 17.81 |
| PCL on nanostructured PMMA | 86.65 |
| *mg of enzyme per 100 mg of polymer | |

Table 5 - Immobilisation of the lipase from *Pseudomonas cepacia* (PCL) on nanostructured polystyrene (PS) (365nm $\varnothing$)

| SAMPLE | ENZYMATIC CHARGE * |
|---|---|
| PCL on amorphous PS | 27.29 |
| PCL on nanostructured PS | 84.46 |
| *mg of enzyme per 100 mg of polymer | |

Table 6 - Immobilisation of the lipase from *Candida rugosa* (CRL) on nanostructured polymethylmethacrylate (PMMA) (309 nm $\varnothing$)

| SAMPLE | ENZYMATIC CHARGE § | UI/mg$_{solid}$ | RESIDUAL ACTIVITY* % |
|---|---|---|---|
| CRL on amorphous PMMA | 28.19 | 0.52 | 43 |
| CRL on nanostructured PMMA | 82.79 | 2.45 | 70 |
| * Free CRL activity - immobilised CRL activity/100 §*mg of enzyme per 100 mg of polymer | | | |

Table 7 - Immobilisation of the lipase from *Candida rugosa* (CRL) on nanostructured polystyrene (PS) (365 nm $\varnothing$)

| SAMPLE | ENZYMATIC CHARGE § | UI/mg$_{solid}$ | RESIDUAL ACTIVITY* % |
|---|---|---|---|
| CRL on amorphous PS | 26.96 | 0.6 | 34 |
| CRL on nanostructured PS | 81.52 | 2.9 | 83 |
| * Free CRL activity- immobilised CRL activity/100 §*mg of enzyme per 100 mg of polymer | | | |

With regard to the studies on the characteristics of nanostructured systems for optics applications (photonic crystals), in particular systematic investigations were conducted to see which chemical-physical factors influence obtaining extended ordered domains. Since the packaging of the particles and obtaining ample crystalline domains is linked to the interactions between particles and between particles and substrate, a systematic study was carried out on the conditions of deposition of PMMA nanospheres (diameter 250 nm).

Particularly studied was the effect of:

■ different deposition techniques (such as spin coating, casting, dipping, evaporation from solution, the Langmuir-Blodget technique);
■ different rates of evaporation of the solvent from the suspension of nanoparticles (by varying deposition temperature and humidity, see Tables 8 and 9);
■ the different ionic force of the suspensions (by varying salt concentration in the suspension, see Table 10);
■ different substrates for the deposition (such as glass, quarts, mica, gold, silica, cadmium behenate; see Table 11).

Table 8 - Ordered domains obtained by varying the deposition temperature in thermostat equipped chamber (RH% 50).

| Temperature (°C) | Domains ($\mu$m$^2$) |
|---|---|
| 2 | 49 |
| 20 | 72 |
| 30 | 80 |
| 40 | 120 |
| 50 | 96 |

Table 9 - Ordered domains obtained by varying humidity at temperature T = 25°C.

| RH% | Domains ($\mu$m$^2$) |
|---|---|
| 6 | 60 |
| 9 | 55 |
| 29 | 81 |

(continued)

| RH% | Domains ($\mu m^2$) |
|---|---|
| 51 | 390 |
| 62 | 252 |
| 75 | 126 |
| 84 | 119 |
| 97 | 85 |

Table 10 - Ordered domains obtained by varying ionic force by adding $MgSO_4$.

| Ionic force | Domains ($\mu m^2$) |
|---|---|
| 1.646 | 483 |
| 0.823 | 480 |
| 0.165 | 352 |
| 0.016 | 275 |
| 0.008 | 264 |
| 0 | 160 |

Table 11- Ordered domains obtained by varying the substrate for the deposition

| Substrate | Domains ($\mu m^2$) |
|---|---|
| glass | 160 |
| graphite | 216 |
| mica | 220 |
| BeCd | 256 |
| Au | 352 |
| Si | 900 |

From these studies, it is apparent that the greater dimensions of the crystalline domains suitable to obtain materials with photonic band-gap are obtained by PMMA deposition on silica substrates (Si), with suspension ionic force of about 2, and at temperature and relative humidity (RH) of 50°C and 50%, respectively.

Production of various morphologies of PPA

PPA, obtained by the synthesis in emulsion described in the bibliography [14], was solubilised in different solvents:

■ THF, which allows to have polymer concentrations of 5 mg/ml; 5 ml of solution were placed in a dialysis bag, then immersed in a crystalliser containing 100 ml of distilled water (v/v solvent-non-solvent ratio = 1/40) at ambient temperature. A yellow precipitate was obtained, whose SEM analysis showed the presence of a small quantity of spheroidal structures, similar in appearance to those produced in the emulsion synthesis; this type of morphology was indicated as no. 1; a similar result was obtained with a solution at lower concentration (0.5 mg/ml) and with a ratio between solvent/non-solvent v/v = 1/20. Most of the polymer obtained in this case had an intermediate morphology between spheroidal pearls and amorphous clots: with the SEM, a highly irregular surface is observed, with a specific area that is definitely greater than the one obtained from the previous test and indicated as morphology no. 2.
■ DMF, producing solutions at different concentration: 0.5mg/ml, 5mg/ml, 10mg/ml. Different solvent / non-solvent ratios and different non solvents were used, conducting the tests also at different temperatures or changing the type of contact with the non solvent (immersion or dripping). Changing the parameters, all 5 different morphologies can be obtained (see table 1). Starting from a saturated solution in DMF, using water as non-solvent and a double dialysis bag (i.e., placing the bag containing the polymer inside a bag with greater diameter, also filled with DMF), to further slow down the process, a sample in the form of spheroidal particles was obtained. Another sample in the form of spheroidal particles was obtained with a single membrane, DMF as solvent and MeOH as non-solvent. In this latter case, by further slowing down the mixing process with the use of a double dialysis membrane, larger particles were obtained than those obtained with a single membrane in the same conditions, but without the presence of amorphous polymer. We stress that the grid structure was revealed, probably thanks to its vast surface area, to be particularly suitable to the use in the immobilisation of molecules with biological activity such as enzymes, drugs, receptors, etc.

■ DMSO, solution concentration of 0.5 and 5 mg/ml. Tests of precipitation from DMSO (always water as non-solvent, but in different ratios) produced complex structures with high surface area, morphology no. 1.

Production of various morphologies of PtDEBP

PtDEBP, obtained by the synthesis in [15], was solubilised in:

■ THF, at solution concentration of 5 mg/ml, using a single membrane, distilled water as non solvent and a v/v solvent / non-solvent ratio = 1/40, at ambient temperature. A yellowish precipitate was obtained, whose SEM analysis showed the presence of a no. 2 type of morphology;

■ DMF, at solution concentration of 1 mg/ml, using a single membrane, acetone as non solvent and a v/v solvent / non-solvent ratio = 1/40, at ambient temperature. A yellowish precipitate was obtained, whose SEM analysis showed the presence of a no. 2 type of morphology, similar to the one obtained from the test with THF/$H_2$O.

Production of various morphologies of PMMA

Commercial PMMA was solubilised in:

■ THF, at solution concentration of 5 or 10 mg/ml, using a single membrane, distilled water as non solvent and a v/v solvent / non-solvent ratio = 1/20 or 1/40, at ambient temperature. A precipitate was obtained, whose SEM analysis showed the presence of: a spongy morphology, no. 3 starting from a concentration of 5 mg/ml and a v/v solvent/non-solvent ratio = 1/20, spheres with morphology no. 5, starting from 10 mg/ml and a v/v solvent / non-solvent ratio = 1/40. In the tests conducted with concentration 10 mg/ml and temperatures from 40°C to 50°C, morphology becomes amorphous, almost a lacquer, both when proceeding by immersion and by dripping of the non-solvent (dripping rate = 1 m/min).

■ DMF, at different solution concentrations of 5 and 10 mg/ml, using a single membrane and different solvent / non-solvent ratios. When distilled water is used as non-solvent and a v/v solvent/non-solvent ratio = 1/20 is used, spongy and tubular morphologies can be obtained, varying polymer concentration in the initial solution. If methanol is used as non-solvent, molten spheres are obtained, i.e. morphology no. 4.

Production of various morphologies of PSt

Commercial PSt was solubilised in:

■ DMF, solution concentration of 5 and 10 mg/ml, with single membrane. Using distilled water as non-solvent, tubular forms and molten spheres can be obtained (morphologies no. 3 and no. 4) varying polymer concentration and solvent/non-solvent ratios. The tests conducted at concentration 10 mg/ml but varying the type of non-solvent, i.e. using methanol and ethanol, also with v/v solvent/non-solvent ratio = 1/20, allowed to obtain morphologies with molten spheres and tubules (morphologies no. 3 and no. 4).

Physical lipase adsorption on polymeric nanospheres of PMMA and PS

After the production of the last two morphologies described (PMMA and PSt), a method was perfected which enables the adsorption of proteins with enzymatic activity, such as the lipases originating from microbial and fungal sources in order to promote their transport.

To an enzymatic solution at predetermined concentration in phosphate buffer pH = 7 ionic force 0.1 is added an appropriate quantity of polymeric material with a ratio (enzyme weight/polymer weight) We/Wp = 1:1. The suspension is subjected to magnetic agitation at 500 rpm for 4 h at the temperature of 25°C. The suspension is then filtered using cellulose acetate filters with Ø of 0.1 nm. The Bradford assay was used to evaluate the concentration of non adsorbed enzyme on the polymeric matrix and hence of the real enzymatic charge on the nanostructured polymer.

Standard assay of tributyrin hydrolysis by lipolytic enzymes

The standard assay of tributyrin hydrolysis by lipase consists of obtaining a reaction mixture constituted by 100 ul of a 50mg/ml solution of enzyme in phosphate buffer pH 7.6, 2.5 ml of phosphate buffer pH 7.6, 0.5 ml of tributyrin. This reaction mixture is incubated for 30 min at the temperature of 37°C in a thermostatic bath with magnetic agitation (600 rpm).
After 30 min the reaction is blocked with 3 ml of acetone:ethanol 1/1 and the free acid is titrated with NaOH 0.1N in the presence of phenolphthalein as indicator.
Enzymatic activity expressed as micromoles of freed acid per minute (UI) is given by the following formula:

$$UI \text{ (ueq acid/min)} = [(A-B)] \times 1000/30$$

A = ml of NaOH used to titrate the sample;
A = ml of NaOH used to titrate the reaction blank.

Bibliography

[1] E. Yablonovitch, Phys. Rev. Lett. 58 (1987) 2059
[2] S. John, Phys. Rev. Lett. 58 (1987) 2486
[3] C. Nguyen, E. Allemann, G. Schwach, E. Doelker, R. Gurny, International Journal of Pharmaceutics 254(1) (2003) 69
[4] M. Koch, A. Falcou, N. Nenov, M. Klapper, K. Mullen, Macromolecular Rapid Communications 22(17) (2001) 1455
[5] W. J. Beek, M. M. Wienk, R. A. J. Janssen, Advanced Materials 16(12) (2004) 1009
[6] X. Wang, M.S. El-Aasser, E.D. Sudol, Macromol. 34 (2001) 7715
[7] W.D. Harkins, J. Am. Chem. Soc. 69 (1947) 1428
[8] C.S. Chern, T.J. Chen, Colloids Surfaces A: Physicochem. Eng. Aspects 138 (1998) 65
[9] W.V. Smith, J. Am. Chem. Soc. 71 (1949) 4077
[10] R. D'Amato, L. Medei, I. Venditti, M. V. Russo, M. Falconieri, Materials Science & Engineering C 23 (2003) 861
[11] I. Capek, S-Y. Lin, T-J. Hsu, C-S Chern, J. Polymer Sci. Part A: Polymer Chemistry 38 (9), (2000) 1477
[12] M. Muller, R. Zentel, T. Maka, S. G. Romanov, C. M. Sotomayor Torres Adv. Mater. 12, (2000) 1499
[13] J.M. Stubbs, D.C. Sundberg, J. Appl. Pol. Sci. 91 (2004) 1538
[14 A. Furlani, C. Napoletano, M. V. Russo, A. Camus, N. Marsich, J. Polym. Sci.: A: Polym. Chem. 27 (1989) 75.
[15] I. Fratoddi, P. Altamura, C. Lo Sterzo, A. Furlani, E. Galassi, A. D'Amico, M.V. Russo, Polymers for Advanced Technologies 13 (2002) 269
[16] M. Müller, R. Zentel, T. Maka, S. G. Romanov, C. M. Sotomayor Torres, Chem. Mater. 12 (2000) 2508
[17] S. Wong, V. Kitaev, G. A. Ozin, J. Am. Chem. Soc. 125 (2003) 15589

**Claims**

1.  A method for controlling the dimensions and the morphology of the nanostructure of at least one polymer, comprising the steps of:

    - exposing, through a semi-permeable membrane that allows the passive transport of solutes, a solution of the polymer, pre-dissolved in a solvent able to dissolve the polymer in substantially complete fashion, to a non-solvent of the polymer itself, in which the non solvent is miscible with the solvent; wherein the temperature, the v/v solvent / non-solvent ratio and the polymer concentration are so selected as to obtain the precipitate polymer in the desired nanostructure;
    - separating the precipitate polymer from the solution.

2.  The method according to claim 1, wherein the semi-permeable membrane is an osmotic membrane.

3. The method according to claim 1, wherein the semi-permeable membrane is a dialysis membrane.

4. The method according to one of the previous claims wherein the exposure is conducted between a solution of the polymer in a solvent able to dissolve the polymer in substantially complete fashion, and the same solvent of the polymer into which the non-solvent of the polymer is gradually added.

5. The method according to claim 1, wherein the polymer is natural or synthetic.

6. The method according to claim 5, wherein the synthetic polymer belongs to classes of polymers obtainable by polymerisation in stages, or by chain polymerisation.

7. The method according to claim 6, wherein the polymer belongs to the following group: Polyphenylacetylene (PPA), Poly(diethynylbiphenylditributylphosphinaPt(II)) (PtDEBP), Polymethylmethacrylate (PMMA), Polystyrene (PSt).

8. The method according to one of the previous claims wherein the solvent and the non-solvent belong to the groups: Alcohols, Ketones, Ethers, Amides, aliphatic, aromatic and chlorinated organic solvents and $H_2O$.

9. The method according to claim 8, wherein the solvent and the non solvent belong to group: Acetone, Methanol, Ethanol, Tetrahydrofuran, Dimethylformamide, Dimethylsulphoxide, Acetonitrile, Toluene, Chloroform, Hexane, $H_2O$.

**Patentansprüche**

1. Verfahren zum Kontrollieren der Dimensionen und der Morphologie der Nanostruktur mindestens eines Polymers, umfassend die folgenden Schritte:

- Belasten, durch eine semipermeable Membran, die den passiven Transport gelöster Stoffe ermöglicht, einer Lösung des Polymers, die in einem Lösemittel vorgelöst ist, das das Polymer im Wesentlichen vollständig auflösen kann, mit einem Nicht-Lösemittel des Polymers selbst, wobei das Nicht-Lösemittel mit dem Lösemittel mischbar ist; wobei die Temperatur, das v/v-Verhältnis von Lösemittel zu Nichtlösemittel und die Polymerkonzentration so gewählt sind, dass das Ausfällungspolymer in der gewünschten Nanostruktur erhalten wird;
- Abtrennen des Ausfällungspolymers von der Lösung.

2. Verfahren nach Anspruch 1, wobei die semipermeable Membran eine osmotische Membran ist.

3. Verfahren nach Anspruch 1, wobei die semipermeable Membran eine Dialysemembran ist.

4. Verfahren nach einem der vorangehenden Ansprüche, wobei die Belastung zwischen einer Lösung des Polymers in einem Lösemittel, das das Polymer im Wesentlichen vollständig auflösen kann, und demselben Lösemittel des Polymers ausgeführt wird, dem das Nicht-Lösemittel des Polymers langsam zugesetzt wird.

5. Verfahren nach Anspruch 1, wobei das Polymer natürlich oder synthetisch ist.

6. Verfahren nach Anspruch 5, wobei das synthetische Polymer zu Klassen von Polymeren gehört, die durch stufenweise Polymerisation oder durch Kettenpolymerisation erhältlich sind.

7. Verfahren nach Anspruch 6, wobei das Polymer zu der folgenden Gruppe gehört: Polyphenylacetylen (PPA), Poly(diethynylbiphenylditributylphosphinaPt(II)) (PtDEBP), Polymethylmethacrylat (PMMA), Polystyrol (Pst).

8. Verfahren nach einem der vorangehenden Ansprüche, wobei das Lösemittel und das Nicht-Lösemittel zu den folgenden Gruppen gehören: Alkohole, Ketone, Ether, Amide, aliphatische, aromatische und chlorinierte organische Lösemittel und $H_2O$.

9. Verfahren nach Anspruch 8, wobei das Lösemittel und das Nicht-Lösemittel zu der folgenden Gruppe gehören: Aceton, Methanol, Ethanol, Tetrahydrofuran, Dimethylformamid, Dimethylsulphoxid, Acetonitril, Toluol, Chloroform, Hexan, $H_2O$.

**Revendications**

1. Procédé pour contrôler les dimensions et la morphologie de la nanostructure d'au moins un polymère, comprenant les étapes consistant à :

   - exposer, au travers d'une membrane semi-perméable qui permet le transport passif de solutés, une solution du polymère, pré-dissous dans un solvant capable de dissoudre le polymère d'une manière essentiellement complète, à un non-solvant du polymère lui-même, dans lequel le non solvant est miscible avec le solvant ; dans lequel la température, le rapport volume/volume du solvant / non-solvant et la concentration du polymère sont sélectionnés de manière telle à obtenir le polymère précipité dans la nanostructure souhaitée ;
   - séparer le polymère précipité de la solution.

2. Procédé selon la revendication 1, dans lequel la membrane semi-perméable est une membrane osmotique.

3. Procédé selon la revendication 1, dans lequel la membrane semi-perméable est une membrane à dialyse.

4. Procédé selon l'une quelconque des revendications précédentes, dans lequel l'exposition est menée entre une solution du polymère dans un solvant capable de dissoudre le polymère d'une manière essentiellement complète, et le même solvant du polymère dans lequel le non-solvant du polymère est ajouté petit-à-petit.

5. Procédé selon la revendication 1, dans lequel le polymère est naturel ou synthétique.

6. Procédé selon la revendication 5, dans lequel le polymère synthétique appartient à des catégories de polymères pouvant être obtenues par une polymérisation en étapes, ou par polymérisation en chaîne.

7. Procédé selon la revendication 6, dans lequel le polymère appartient au groupe suivant : polyphénylacétylène (PPA), poly(diéthynylbiphénylditributylphosphinaPt(II)) (PtDEBP), polyméthylméthacrylate (PMMA), polystyrène (PSt).

8. Procédé selon l'une quelconque des revendications précédentes, dans lequel le solvant et le non-solvant appartiennent aux groupes des : alcools, cétones, éthers, amides, solvants organiques aliphatiques, aromatiques et chlorés et $H_2O$.

9. Procédé selon la revendication 8, dans lequel le solvant et le non-solvant appartiennent au groupe : de l'acétone, du méthanol, de l'éthanol, du tétrahydrofurane, du diméthylformamide, du diméthylsulfoxyde, de l'acétonitrile, du toluène, du chloroforme, de l'hexane, de l'$H_2O$.

# Fig.1

PtDEBP

PMMA

PPA

PSt

Fig.2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **E. YABLONOVITCH.** *Phys. Rev. Lett.,* 1987, vol. 58, 2059
- **S. JOHN.** *Phys. Rev. Lett.,* 1987, vol. 58, 2486
- **C. NGUYEN ; E. ALLEMANN ; G. SCHWACH ; E. DOELKER ; R. GURNY.** *International Journal of Pharmaceutics,* 2003, vol. 254 (1), 69
- **M. KOCH ; A. FALCOU ; N. NENOV ; M. KLAPPER ; K. MULLEN.** *Macromolecular Rapid Communications,* 2001, vol. 22 (17), 1455
- **W. J. BEEK ; M. M. WIENK ; R. A. J. JANSSEN.** *Advanced Materials,* 2004, vol. 16 (12), 1009
- **X. WANG ; M.S. EL-AASSER ; E.D. SUDOL.** *Macromol.,* 2001, vol. 34, 7715
- **W.D. HARKINS.** *J. Am. Chem. Soc.,* 1947, vol. 69, 1428
- **C.S. CHERN ; T.J. CHEN.** *Colloids Surfaces A: Physicochem. Eng. Aspects,* 1998, vol. 138, 65
- **W.V. SMITH.** *J. Am. Chem. Soc.,* 1949, vol. 71, 4077
- **R. D'AMATO ; L. MEDEI ; I. VENDITTI ; M. V. RUSSO ; M. FALCONIERI.** *Materials Science & Engineering C,* 2003, vol. 23, 861
- **I. CAPEK ; S-Y. LIN ; T-J. HSU ; C-S CHERN.** *J. Polymer Sci. Part A: Polymer Chemistry,* 2000, vol. 38 (9), 1477
- **M. MULLER ; R. ZENTEL ; T. MAKA ; S. G. ROMANOV ; C. M. SOTOMAYOR TORRES.** *Adv. Mater.,* 2000, vol. 12, 1499
- **J.M. STUBBS ; D.C. SUNDBERG.** *J. Appl. Pol. Sci.,* 2004, vol. 91, 1538
- **A. FURLANI ; C. NAPOLETANO ; M. V. RUSSO ; A. CAMUS ; N. MARSICH.** *J. Polym. Sci.: A: Polym. Chem.,* 1989, vol. 27, 75
- **I. FRATODDI ; P. ALTAMURA ; C. LO STERZO ; A. FURLANI ; E. GALASSI ; A. D'AMICO ; M.V. RUSSO.** *Polymers for Advanced Technologies,* 2002, vol. 13, 269
- **M. MÜLLER ; R. ZENTEL ; T. MAKA ; S. G. ROMANOV ; C. M. SOTOMAYOR TORRES.** *Chem. Mater.,* 2000, vol. 12, 2508
- **S. WONG ; V. KITAEV ; G. A. OZIN.** *J. Am. Chem. Soc.,* 2003, vol. 125, 15589